# EUROPEAN PATENT SPECIFICATION

(11) **EP 2 432 388 B1**
(45) Date of publication and mention of the grant of the patent: **02.09.2015**
(21) Application number: 10723867.7
(22) Date of filing: 19.05.2010
(51) Int. Cl.: A61B 5/06, A61B 19/00, A61N 1/05

(54) **SYSTEM FOR CARDIAC LEAD PLACEMENT**
SYSTEM ZUR POSITIONIERUNG VON HERZELEKTRODEN
SYSTÈME POUR PLACEMENT DE DÉRIVATION CARDIAQUE

(30) Priority: 19.05.2009 US 468140
(43) Date of publication of application: 28.03.2012
(73) Proprietor: Medtronic, Inc, Minneapolis, Minnesota 55432 (US)
(72) Inventor: WENGER, William, K., St. Paul, MN 55416 (US)
(74) Representative: Hughes, Andrea Michelle
(86) International application number: PCT/US2010/035396
(87) International publication number: WO 2010/135420

(56) References cited:
- EP-A1- 1 421 913
- EP-A1- 1 504 713
- EP-A2- 1 523 951
- EP-A2- 1 743 591
- US-A1- 2005 085 715
- US-A1- 2006 084 867
- US-A1- 2007 249 911
- US-A1- 2010 030 061
- US-A1- 2010 030 063
- US-B1- 6 868 291

## Description

### INTRODUCTION

The human anatomy includes many types of tissue that can either voluntarily or involuntarily, perform certain functions. However, after disease or injury, certain tissues may no longer operate within general anatomical norms. For example, after disease, injury, age, or combinations thereof, the heart muscle may begin to experience certain failures or deficiencies. Some of these failures or deficiencies can be corrected or treated with implantable medical devices (IMDs). These devices can include implantable pulse generator (IPG) devices, pacemakers, implantable cardioverter-defibrillator (ICD) devices, cardiac resynchronization therapy defibrillator devices, or combinations thereof.

One of the main portions of the IMD can include a lead that is directly connected to tissue to be affected by the IMD. The lead can include a tip portion that is directly connected to the anatomical tissue, such as a muscle bundle, and a lead body that connects to the device body or therapeutic driving device. It is generally known that the device body or case portion can be implanted in a selected portion of the anatomical structure, such as in a chest or abdominal wall, and the lead can be inserted through various venous portions so that the tip portion can be positioned at the selected position near or in the muscle group.

The IMDs are implantable devices that may require the use of imaging devices for implantation. The imaging devices can include fluoroscopes that expose a patient and a surgeon to ionizing radiation. In addition, the use of the imaging device can require time for acquiring image data and understanding the images from the image data.

EP 1054713 relates to systems and methods for using one or more medical images to assist in navigating an instrument through internal body structures.

### SUMMARY

The present disclosure relates to implantable medical devices (IMD)s, in particular to a system and method for a cardiac lead system having electromagnetic placement confirmation.

In this regard, provided is a system for determining a location of a cardiac lead within an anatomy. The system can include a cardiac lead for insertion into an anatomy, which can define at least one conduit. The system can also include a confirmation member that can be positionable within the at least one conduit and movable relative to the cardiac lead. The system can include at least one tracking device, which can be coupled to the confirmation member, and a tracking system that can track a position of the at least one tracking device relative to the anatomy. The system can also include a navigation system that determines a position of the confirmation member relative to the anatomy based on the position of the at least one tracking device. The navigation system can also determine a position and a shape of the cardiac lead within the anatomy based on the position of the confirmation member.

Further provided is a method for determining a location of a cardiac lead within an anatomy. The method can include coupling at least one tracking device to a flexible instrument, and inserting the flexible instrument into at least one conduit defined in the cardiac lead. The method can include moving the flexible instrument within the cardiac lead, and tracking the at least one tracking device relative to the anatomy. The method can also include determining, based on the tracking of the at least one tracking device, a position of flexible instrument relative to the anatomy and determining, based on the position of the flexible instrument, a position of the cardiac lead relative to the anatomy. The method can include displaying the position of the cardiac lead as an icon superimposed onto an image of the anatomy.

In addition, a method for determining a location of a cardiac lead within an anatomy is provided. The method can include inserting a cardiac lead into an anatomy that defines at least one conduit, and coupling at least one electromagnetic tracking device to a distal end of a flexible tubular member. The method can also include inserting at least the distal end of the flexible tubular member into the at least one conduit of the cardiac lead, and moving the flexible tubular member within the at least one conduit of the cardiac lead. The method can include tracking the at least one electromagnetic tracking device relative to the anatomy with an electromagnetic tracking system, and determining, based on the tracking of the at least one electromagnetic tracking device, a position of flexible tubular member relative to the anatomy. The method can include determining, based on the position of the flexible tubular member, a position of the cardiac lead relative to the anatomy, and displaying the position of the cardiac lead as an icon superimposed onto an image of the anatomy.

Further areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.
FIG. 1 is a diagram of a navigation system for performing a surgical procedure on a patient according to various exemplary embodiments of the present disclosure;
FIG. 2 is a simplified schematic illustration of an exemplary cardiac lead including a confirmation member according to various teachings;
FIG. 3 is a cross-sectional schematic illustration of the cardiac lead of FIG. 2, taken along line 3-3 of FIG. 2;
FIG. 4 is a schematic illustration of an exemplary confirmation member for use with the cardiac lead of FIG. 2 according to various teachings;
FIG. 5 is a cross-sectional schematic illustration of an exemplary confirmation member for use with the cardiac lead of FIG. 2, taken along line 5-5 of FIG. 2;
FIG. 6 is a simplified block diagram illustrating the navigation system of FIG. 1;
FIG. 7 is a dataflow diagram illustrating a control system performed by a control module associated with the navigation system of FIG. 1; and
FIG. 8 is a flowchart illustrating a control method performed by the control module.

### DETAILED DESCRIPTION

The following description is merely exemplary in nature and is not intended to limit the present disclosure, application, or uses. It should be understood that throughout the drawings, corresponding reference numerals indicate like or corresponding parts and features. As indicated above, the present teachings are directed towards providing a system and method for confirming the placement of a cardiac lead. It should be noted, however, that the present teachings could be applicable to any appropriate procedure in which it is desirable to determine a position of a cannulated structure within an anatomy using an electromagnetic navigation system. Therefore, it will be understood that the following discussions are not intended to limit the scope of the appended claims. Further, as used herein, the term "module" can refer to an application specific integrated circuit (ASIC), an electronic circuit, a processor (shared, dedicated, or group) and memory that executes one or more software or firmware programs, a combinational logic circuit, and/or other suitable software, firmware programs or components that provide the described functionality. Therefore, it will be understood that the following discussions are not intended to limit the scope of the appended claims.

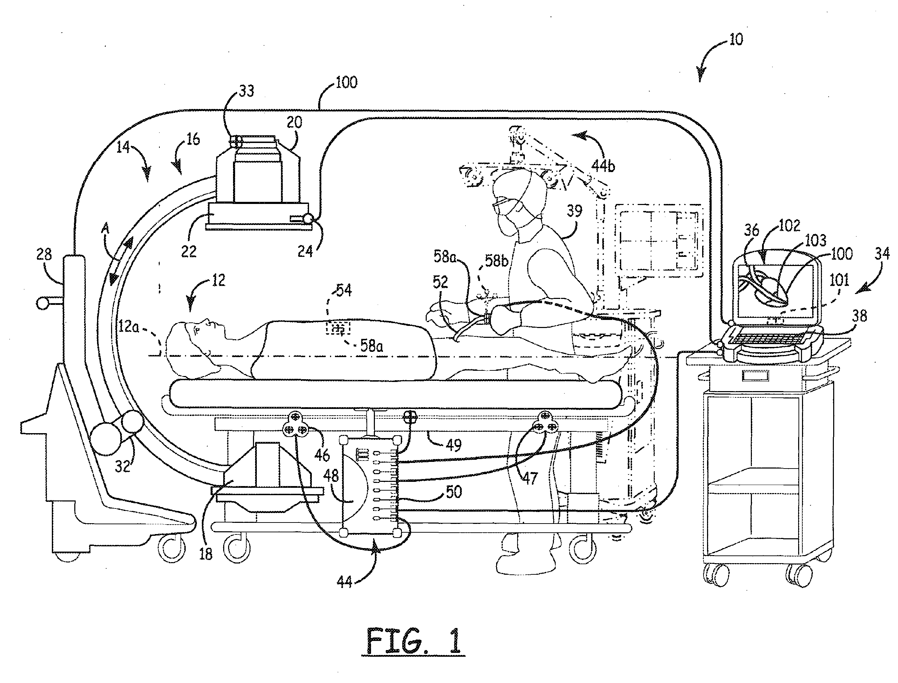

FIG. 1 is a diagram illustrating an overview of a navigation system 10 that can be used for various procedures. The navigation system 10 can be used to track the location of an implant, such as a spinal implant or orthopedic implant, relative to a patient 12. Also the navigation system 10 can track the position and orientation of various instruments. It should further be noted that the navigation system 10 may be used to navigate any type of instrument, implant, or delivery system, including: guide wires, arthroscopic systems, cardiac leads, orthopedic implants, spinal implants, deep-brain stimulator (DBS) probes, etc. Moreover, these instruments may be used to navigate or map any region of the body. The navigation system 10 and the various instruments may be used in any appropriate procedure, such as one that is generally minimally invasive, arthroscopic, percutaneous, stereotactic, or an open procedure.

The navigation system 10 may include an imaging device 14 that is used to acquire pre-, intra-, or post-operative or real-time image data of a patient 12. Alternatively, various imageless systems can be used or images from atlas models can be used to produce patient images, such as those disclosed in U.S. Patent Pub. No. 2005-0085714, filed 10/16/2003, entitled "Method And Apparatus For Surgical Navigation Of A Multiple Piece Construct for Implantation. The imaging device 14 can be, for example, a fluoroscopic x-ray imaging device that may be configured as an O-arm™ or a C-arm 16 having an x-ray source 18, an x-ray receiving section 20, an optional calibration and tracking target 22 and optional radiation sensors 24. It will be understood, however, that patient image data can also be acquired using other imaging devices, such as those discussed above and herein.

In operation, the imaging device 14 generates x-rays from the x-ray source 18 that propagate through the patient 12 and calibration and/or tracking target 22, into the x-ray receiving section 20. This allows real-time visualization of the patient 12 and radio-opaque instruments, via the X-rays. In the example of FIG. 1, a longitudinal axis 12a of the patient 12 is substantially in line with a mechanical rotational axis 32 of the C-arm 16. This can enable the C-arm 16 to be rotated relative to the patient 12, allowing images of the patient 12 to be taken from multiple directions or about multiple planes. An example of a fluoroscopic C-arm X-ray device that may be used as the optional imaging device 14 is the "Series 9600 Mobile Digital Imaging System," from GE Healthcare (formerly OEC Medical Systems, Inc.) of Salt Lake City, Utah. Other exemplary fluoroscopes include bi-plane fluoroscopic systems, ceiling fluoroscopic systems, cath-lab fluoroscopic systems, fixed C-arm fluoroscopic systems, isocentric C-arm fluoroscopic systems, 3D fluoroscopic systems, etc. An exemplary O-arm™ imaging device is available from Medtronic Navigation, Inc. of Littleton, MA.

When the x-ray source 18 generates the x-rays that propagate to the x-ray receiving section 20, the radiation sensors 24 can sense the presence of radiation, which is forwarded to an imaging device controller 28, to identify whether or not the imaging device 14 is actively imaging. This information can also be transmitted to a coil array controller 48, further discussed herein.

The imaging device controller 28 can capture the x-ray images received at the x-ray receiving section 20 and store the images for later use. Multiple two-dimensional images taken by the imaging device 14 may also be captured and assembled by the imaging device controller 28 to provide a larger view or image of a whole region of the patient 12, as opposed to being directed to only a portion of a region of the patient 12. For example, multiple image data of a leg of the patient 12 may be appended together to provide a full view or complete set of image data of the leg that can be later used to follow contrast agent, such as Bolus tracking. The imaging device controller 28 may also be separate from the C-arm 16 and/or control the rotation of the C-arm 16. For example, the C-arm 16 can move in the direction of arrow A or rotate about the longitudinal axis 12a of the patient 12, allowing anterior or lateral views of the patient 12 to be imaged. Each of these movements involves rotation about a mechanical rotational axis 32 of the C-arm 16. The movements of the imaging device 14, such as the C-arm 16 can be tracked with a tracking device 33.

While the imaging device 14 is shown in FIG. 1 as a C-arm 16, any other alternative 2D, 3D or 4D imaging modality may also be used. For example, any 2D, 3D or 4D imaging device, such as an O-arm™ imaging device, isocentric fluoroscopy, bi-plane fluoroscopy, ultrasound, computed tomography (CT), multi-slice computed tomography (MSCT), magnetic resonance imaging (MRI), high frequency ultrasound (HFU), positron emission tomography (PET), optical coherence tomography (OCT), intra-vascular ultrasound (IVUS), ultrasound, intra-operative CT or MRI may also be used to acquire 2D, 3D or 4D pre- or post-operative and/or real-time images or patient image data 100 of the patient 12. For example, an intra-operative MRI system, may be used such as the PoleStar® MRI system sold by Medtronic, Inc.

In addition, image datasets from hybrid modalities, such as positron emission tomography (PET) combined with CT, or single photon emission computer tomography (SPECT) combined with CT, could also provide functional image data superimposed onto anatomical data to be used to confidently reach target sites within the patient 12. It should further be noted that the imaging device 14, as shown in FIG. 1, provides a virtual bi-plane image using a single-head C-arm fluoroscope as the imaging device 14 by simply rotating the C-arm 16 about at least two planes, which could be orthogonal planes, to generate two-dimensional images that can be converted to three-dimensional volumetric images. By acquiring images in more than one plane, an icon 103 representing the location of an instrument 52, such as an impacter, stylet, reamer driver, taps, drill, deep-brain stimulator (DBS) probes, cardiac leads, catheter, balloon catheter, basket catheter, or other instrument, or implantable devices introduced and advanced in the patient 12, may be superimposed in more than one view and included in image data 102 displayed on a display 36, as will be discussed.

If the imaging device 14 is employed, patient image data 100 can be forwarded from the imaging device controller 28 to a navigation computer and/or processor or workstation 34. It will also be understood that the patient image data 100 is not necessarily first retained in the imaging device controller 28, but may also be directly transmitted to the workstation 34. The workstation 34 can include the display 36, a user input device 38 and a control module 101. The workstation 34 can also include or be connected to an image processor, navigation processor, and memory to hold instruction and data. The workstation 34 can provide facilities for displaying the patient image data 100 as an image on the display 36, saving, digitally manipulating, or printing a hard copy image of the received patient image data 100.

The user input device 38 can comprise any device that can enable a user to interface with the workstation 34, such as a touchpad, touch pen, touch screen, keyboard, mouse, wireless mouse, air mouse, joystick, or a combination thereof. The user input device 38 allows a physician or user 39 to provide inputs to control the imaging device 14, via the imaging device controller 28, adjust the display settings of the display 36, or control a tracking system 44, as further discussed herein.

The control module 101 can determine the location of a tracking device 58 with respect to the patient space, and can determine a position of the instrument 52 in the patient space. The control module 101 can also determine a shape of the instrument 52 relative to the patient space, and can output image data 102 to the display 36. The image data 102 can include the icon 103 that provides an indication of a location of the instrument 52 with respect to the patient space, illustrated on the patient image data 100, as will be discussed herein.

With continuing reference to FIG. 1, the navigation system 10 can further include the electromagnetic navigation or tracking system 44 that includes a localizer, such as a first coil array 46 and/or second coil array 47, the coil array controller 48, a navigation probe interface 50, a device or instrument 52, a patient tracker or first reference frame or dynamic reference frame (DRF) 54 and one or more tracking devices 58. Other tracking systems can include an optical tracking system 44b, for example the StealthStation® Treon® and the StealthStation® Tria® both sold by Medtronic Navigation, Inc. Further, other tracking systems can be used that include acoustic, radiation, radar, infrared, etc., or hybrid systems such as a system that includes components of both an electromagnetic and optical tracking system, etc. Moreover, a position sensing unit could be employed to determine a position of the instrument 52 relative to the anatomy. An exemplary position sensing unit can comprise the LocaLisa® Intracardiac Navigation System, which is sold by Medtronic, Inc. of Minneapolis, MN. Additionally, the position sensing unit could comprise the position sensing unit described in U.S. Patent Serial No. 12/117,537, entitled "Method and apparatus for Mapping a Structure" and published as US 2009/264,752 or the position sensing unit described in U.S. Patent Serial No. 12/117,549, entitled "Method and Apparatus for Mapping a Structure" and published as US 2009/262,992. In the case of an electromagnetic tracking system 44, the instrument 52 and the DRF 54 can each include tracking device(s) 58.

The tracking device 58 or any appropriate tracking device as discussed herein, can include both a sensor, a transmitter, or combinations thereof and can be indicated by the reference numeral 58. Further, the tracking device 58 can be wired or wireless to provide a signal or emitter or receive a signal from a system. For example, an electromagnetic tracking device 58a can include one or more electromagnetic coil, such as a tri-axial coil, to sense a field produced by the localizing coil array 46 or 47. One will understand that the tracking device(s) 58 can receive a signal, transmit a signal, or combinations thereof to provide information to the navigation system 10, which can be used to determine a location of the tracking device 58. The navigation system 10 can determine a position of the instrument 52 and the DRF 54 based on the location of the tracking device(s) 58 to allow for accurate navigation relative to the patient 12 in the patient space.

With regard to the optical localizer or tracking system 44b, the optical tracking system 44b can transmit and receive an optical signal, or combinations thereof. An optical tracking device 58b can be interconnected with the instrument 52, or other devices such as the DRF 54. As generally known, the optical tracking device 58b can reflect, transmit or receive an optical signal to/from the optical localizer or tracking system 44b that can be used in the navigation system 10 to navigate or track various elements. Therefore, one skilled in the art will understand, that the tracking device(s) 58 can be any appropriate tracking device to work with any one or multiple tracking systems.

The coil arrays 46, 47 can transmit signals that are received by the tracking device(s) 58. The tracking device(s) 58 can then transmit or receive signals based upon the transmitted or received signals from or to the coil arrays 46, 47. The coil arrays 46, 47 are shown attached to the operating table 49. It should be noted, however, that the coil arrays 46, 47 can also be positioned at any other location, as well and can also be positioned in the items being navigated. The coil arrays 46, 47 include a plurality of coils that are each operable to generate distinct electromagnetic fields into the navigation region of the patient 12, which is sometimes referred to as patient space. Representative electromagnetic systems are set forth in U.S. Patent No. 5,913,820, entitled "Position Location System," issued June 22, 1999 and U.S. Patent No. 5,592,939, entitled "Method and System for Navigating a Catheter Probe," issued January 14, 1997,

in addition, representative electromagnetic systems can include the AXIEM™ electromagnetic tracking system sold by Medtronic Navigation, Inc. The coil arrays 46, 47 can be controlled or driven by the coil array controller 48. The coil array controller 48 can drive each coil in the coil arrays 46, 47 in a time division multiplex or a frequency division multiplex manner. In this regard, each coil can be driven separately at a distinct time or all of the coils can be driven simultaneously with each being driven by a different frequency. Upon driving the coils in the coil arrays 46, 47 with the coil array controller 48, electromagnetic fields are generated within the patient 12 in the area where the medical procedure is being performed, which is again sometimes referred to as patient space. The electromagnetic fields generated in the patient space induce currents in a tracking device(s) 58 positioned on or in the instrument 52 and DRF 54. These induced signals from the instrument 52 and DRF 54 are delivered to the navigation probe interface 50 and can be subsequently forwarded to the coil array controller 48.

In addition, the navigation system 10 can include a gating device or an ECG or electrocardiogram triggering device, which is attached to the patient 12, via skin electrodes, and in communication with the coil array controller 48. Respiration and cardiac motion can cause movement of cardiac structures relative to the instrument 52, even when the instrument 52 has not been moved. Therefore, patient image data 100 can be acquired from the imaging device 14 based on a time-gated basis triggered by a physiological signal or a physiological event. For example, the ECG or EGM signal may be acquired from the skin electrodes or from a sensing electrode included on the instrument 52 or from a separate reference probe (not shown). A characteristic of this signal, such as an R-wave peak or P-wave peak associated with ventricular or atrial depolarization, respectively, may be used as a reference of a triggering physiological event for the coil array controller 48 to drive the coils in the coil arrays 46, 47. This reference of a triggering physiological event may also be used to gate or trigger image acquisition during the imaging phase with the imaging device 14. By time-gating the image data 102 and/or the navigation data, the icon 103 of the location of the instrument 52 in image space relative to the patient space at the same point in the cardiac cycle may be displayed on the display 36. Further detail regarding the time-gating of the image data and/or navigation data can be found in U.S. Patent Pub. Application No. 2004-0097806, entitled "Navigation System for Cardiac Therapies," filed November 19, 2002.

The navigation probe interface 50 may provide the necessary electrical isolation for the navigation system 10. The navigation probe interface 50 can also include amplifiers, filters and buffers to directly interface with the tracking device(s) 58 in the instrument 52 and DRF 54. Alternatively, the tracking device(s) 58, or any other appropriate portion, may employ a wireless communications channel, such as that disclosed in U.S. Patent No. 6,474,341, entitled "Surgical Communication Power System," issued November 5, 2002, as opposed to being coupled directly to the navigation probe interface 50.

The instrument 52 may be any appropriate instrument, such as an instrument for preparing a portion of the patient 12, an instrument for treating a portion of the patient 12 or an instrument for positioning an implant, as will be discussed herein. The DRF 54 of the tracking system 44 can be coupled to the navigation probe interface 50. The DRF 54 may be coupled to a first portion of the anatomical structure of the patient 12 adjacent to the region being navigated so that any movement of the patient 12 is detected as relative motion between the coil arrays 46, 47 and the DRF 54. For example, the DRF 54 can be adhesively coupled to the patient 12, however, the DRF 54 could also be mechanically coupled to the patient 12, if desired. The DRF 54 may include any appropriate tracking device(s) 58 used by the navigation system 10. Therefore, the DRF 54 can include an optical tracking device or acoustic, etc. If the DRF 54 is used with an electromagnetic tracking device 58a, it can be configured as a pair of orthogonally oriented coils, each having the same centerline or may be configured in any other non-coaxial or co-axial coil configurations, such as a tri-axial coil configuration (not specifically shown).

Briefly, the navigation system 10 operates as follows. The navigation system 10 creates a translation map between all points in the radiological image generated from the imaging device 14 in image space and the corresponding points in the anatomical structure of the patient 12 in patient space. After this map is established, whenever a tracked instrument, such as the instrument 52 is used, the workstation 34 in combination with the coil array controller 48 and the imaging device controller 28 uses the translation map to identify the corresponding point on the pre-acquired image or atlas model, which is displayed on display 36. This identification is known as navigation or localization. The icon 103 representing the localized point or instruments 52 can be shown as image data 102 on the display 36.

To enable navigation, the navigation system 10 must be able to detect both the position of the anatomical structure of the patient 12 and the position of the instrument 52. Knowing the location of these two items allows the navigation system 10 to compute and display the position of the instrument 52 in relation to the patient 12 on the display 36. The tracking system 44 can be employed to track the instrument 52 and the anatomical structure simultaneously.

The tracking system 44, if using an electromagnetic tracking assembly, essentially works by positioning the coil arrays 46, 47 adjacent to the patient space to generate a low-energy electromagnetic field generally referred to as a navigation field. Because every point in the navigation field or patient space is associated with a unique field strength, the tracking system 44 can determine the position of the instrument 52 by measuring the field strength at the tracking device 58 location. The DRF 54 can be fixed to the patient 12 to identify a location of the patient 12 in the navigation field. The tracking system 44 can continuously recompute the relative position of the DRF 54 and the instrument 52 during localization and relate this spatial information to patient registration data to enable image guidance of the instrument 52 within and/or relative to the patient 12.

Patient registration is the process of determining how to correlate the position of the instrument 52 relative to the patient 12 to the position on the diagnostic or pre-acquired images. To register the patient 12, a physician or user 39 may use point registration by selecting and storing particular points from the pre-acquired images and then touching the corresponding points on the anatomical structure of the patient 12 with a pointer probe. The navigation system 10 analyzes the relationship between the two sets of points that are selected and computes a match, which correlates every point in the patient image data 100 with its corresponding point on the anatomical structure of the patient 12 or the patient space, as discussed herein. The points that are selected to perform registration are the fiducial markers, such as anatomical landmarks. Again, the landmarks or fiducial markers are identifiable on the images and identifiable and accessible on the patient 12. The fiducial markers can be artificial markers that are positioned on the patient 12 or anatomical landmarks that can be easily identified in the patient image data 100. The artificial landmarks, such as the fiducial markers, can also form part of the DRF 54, such as those disclosed in U.S. Patent No. 6,381,485, entitled "Registration of Human Anatomy Integrated for Electromagnetic Localization," issued April 30, 2002.

The navigation system 10 may also perform registration using anatomic surface information or path information as is known in the art. The navigation system 10 may also perform 2D to 3D registration by utilizing the acquired 2D images to register 3D volume images by use of contour algorithms, point algorithms or density comparison algorithms, as is known in the art. An exemplary 2D to 3D registration procedure, is set forth in U.S. Patent Serial No. 10/644,680, entitled "Method and Apparatus for Performing 2D to 3D Registration," filed on August 20, 2003 and published as US 7,570,791.

In order to maintain registration accuracy, the navigation system 10 continuously tracks the position of the patient 12 during registration and navigation. This is because the patient 12, DRF 54 and coil arrays 46, 47 may all move with respect to one another during the procedure, even when this movement is not desired. Alternatively the patient 12 may be held immobile once the registration has occurred, such as with a head frame (not shown). Therefore, if the navigation system 10 did not track the position of the patient 12 or area of the anatomical structure, any patient movement after image acquisition would result in inaccurate navigation within that image. The DRF 54 allows the tracking system 44 to register and track the anatomical structure. Because the DRF 54 can be coupled to the patient 12, any movement of the anatomical structure of the patient 12 or the coil arrays 46, 47 can be detected as the relative motion between the coil arrays 46, 47 and the DRF 54. Both the relative motion of the coil arrays 46, 47 and the DRF 54 can be communicated to the coil array controller 48, via the navigation probe interface 50, which can update the registration correlation to thereby maintain accurate navigation.

The navigation system 10 can be used according to any appropriate method or system. For example, pre-acquired images, atlas or 3D models may be registered relative to the patient 12 and the patient space. Generally, the navigation system 10 allows the images on the display 36 to be registered and to accurately display the real time location of the various instruments, such as the instrument 52, and other appropriate items, such as DRF 54. In addition, the DRF 54 may be used to ensure that any planned or unplanned movement of the patient 12 or the coil arrays 46, 47 can be determined and used to correct the image data 102 on the display 36.

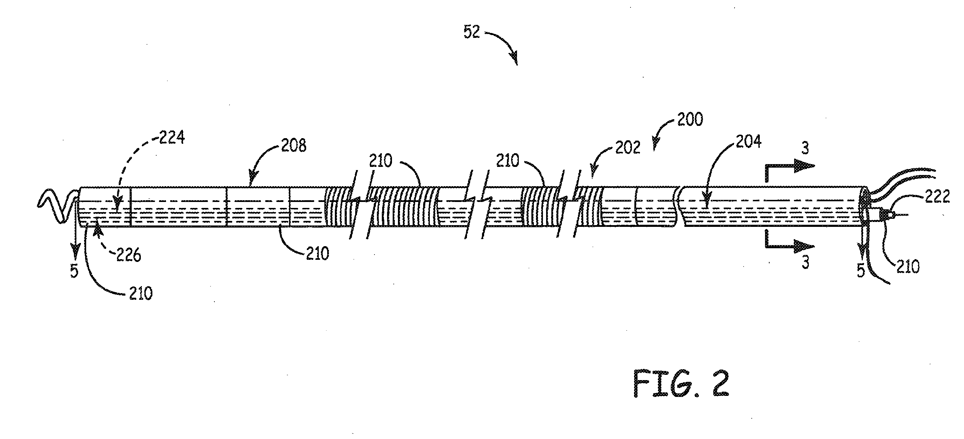

Referring now to FIGS. 1, 2 and 2A, an instrument 52 is shown for use with the tracking system 44. In this case, the instrument 52 comprises an elongated flexible body, such as a cardiac lead system 200. Although a cardiac lead system 200 will be described and illustrated herein, it should be understood that the instrument 52 could comprise any suitable instrument, such as, a catheter, a basket catheter, a balloon catheter, a cardiac lead, guidewire, sheath, endoscope, ablation catheter, arthroscopic instruments, orthopedic instruments, spinal instruments, trocars, deep-brain stimulator (DBS) probes, drug delivery instruments, mapping catheter, etc. Thus, it will be understood that the illustration of the cardiac lead system 200 as the instrument 52 is merely exemplary. Generally, the cardiac lead system 200 can include a lead 202 and a confirmation member 204. The lead 202 can be implanted into an anatomical structure, and the confirmation member 204 can cooperate with the navigation system 10 to ensure that the lead 202 is properly placed within the anatomy.

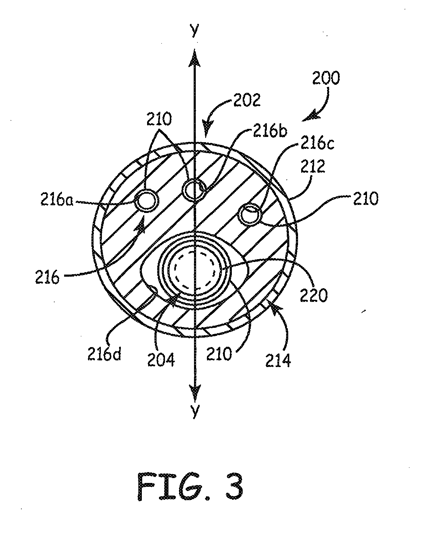

The lead 202 can be coupled to and in communication with a suitable ICD, and can be implanted into an anatomical structure, such as a heart. Generally, the lead 202 can both sense the electrical activity of the heart and can also deliver electrical energy to pace the heart. As the lead 202 can comprise any suitable cardiac lead, such as a SPRINT QUATTRO SECURE™ cardiac lead commercially available from Medtronic, Inc. of Minneapolis, MN, the lead 202 will not be discussed in great detail herein. Briefly, however, the lead 202 can include a body 208 and least one electrode assembly 210. The body 208 can serve to protect, carry and guide the at least one electrode assembly 210 through the anatomical structure. With additional reference to FIG. 3, the body 208 can include an overlay 212 and a multilumen member 214. The overlay 212 can comprise any suitable biocompatible material, such as a biocompatible polymer, and can generally be composed of polyurethane. The overlay 212 can be disposed over the multilumen member 214.

With continued reference to FIG. 3, the multilumen member 214 can define at least one conduit 216 for each of the at least one electrode assembly 210 associated with the lead 202. Thus, in one example, the multilumen member 214 can comprise a first conduit 216a, a second conduit 216b, a third conduit 216c and a fourth conduit 216d. In this example, the first conduit 216a, second conduit 216b and third conduit 216c can have a diameter that may be smaller than a diameter of the fourth conduit 216d. Typically, the first conduit 216a, second conduit 216b, third conduit 216c and fourth conduit 216d can be positioned within the multilumen member 214 such that the multilumen member 214 can be symmetric with respect to an axis Y. The conduits 216 can each receive at least a portion of the electrode assemblies 210.

The at least one electrode assembly 210 can sense the electrical activity of the heart and/or can deliver electrical energy to pace the heart, as is generally known. In this example, the at least one electrode assembly 210 can include four electrode assemblies 210. It should be noted, however, that while the lead 202 is illustrated and described herein as including four electrode assemblies 210a-d in FIGS. 2 and 3, the lead 202 may have any number of electrode assemblies 210. A portion of each of the electrode assemblies 210 can pass through the conduits 216 to enable electrical communication along the lead 202. Generally, the fourth conduit 216d can cooperate with the fourth electrode assembly 210d to define a guide channel 220. The guide channel 220 can be sized to enable receipt of a guide wire therethrough, which can be used to direct or guide the lead 202 to the desired position in the anatomy. The guide channel 220 can also receive the confirmation member 204.

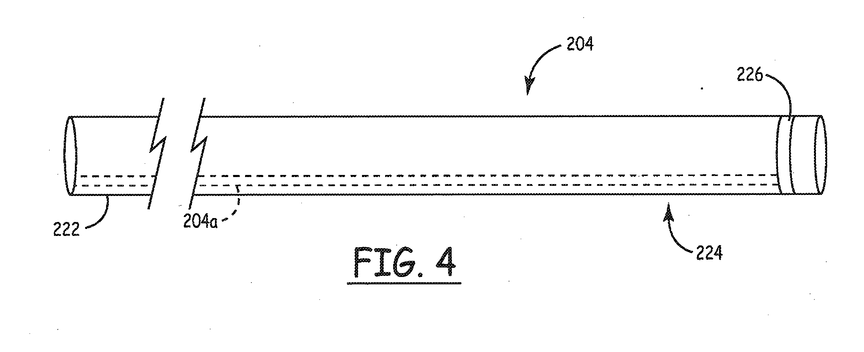

With reference to FIG. 4, the confirmation member 204 can include a proximal end 222, a distal end 224 and at least one tracking device 226. In one example, the confirmation member 204 can comprise an elongated tubing member, which can be at least partially cannulated, and can optionally define a lumen 204a, to enable a portion of the tracking device 226 to pass therethrough, as will be discussed. In this example, the confirmation member 204 can comprise a polymeric tubing member, which can be comprised of any suitable polymeric material, such as polytetrafluoroethylene (PTFE), fluorinated ethylene propylene (FEP), perfluoroalkoxy (PFA), ethylene tetrafluoroethylene (ETFE), etc.

The proximal end 222 can generally extend outside of the anatomical structure of the patient 12 when the confirmation member 204 is used during the surgical procedure (FIG. 2). In some cases, the proximal end 222 can include a graspable portion, to enable the surgeon to manipulate or direct the movement of the distal end 224 of the confirmation member 204 within the anatomical structure. With reference to FIG. 4, the distal end 224 can be opposed from the proximal end 222. The tracking device 226 can be coupled to the distal end 224.

The tracking device 226 can comprise any suitable tracking device 58 that can be tracked by the tracking system 44, such as the electromagnetic tracking device 58a or the optical tracking device 58b, however, it should be understood that that tracking device 226 could comprise any suitable device capable of indicating a position and/or orientation of the confirmation member 204. If the tracking device 226 comprises an electromagnetic tracking device 58a, then one or more wires can pass through the confirmation member 204 to enable the tracking device 226 to communicate with the navigation probe interface 50. It should be understood, that the tracking device 226 could also comprise a wireless electromagnetic tracking device, if desired.

Generally, the tracking device 226 can be fixed to the confirmation member 204 at a known location and can be fixed such that the tracking device 226 does not substantially move relative to the confirmation member 204. As the tracking device 226 can be fixed to a portion of the confirmation member 204, the tracking device 226 can provide a location and/or orientation of the portion of the confirmation member 204 in the patient space substantially in real-time.

It should also be noted that the tracking device 226 could also comprise at least one object that is responsive to the imaging device 14 to generate a signal, such as a radio-opaque marker. If the tracking device 226 is a radio-opaque marker, then the imaging device 14 can be used to track the position of the portion of the confirmation member 204 coupled to the tracking device 226. If the tracking device 226 comprises a radio-opaque marker, then the tracking device 226 can be coupled to an interior surface of the confirmation member 204, or could be secured between one or more layers that comprise the confirmation member 204.

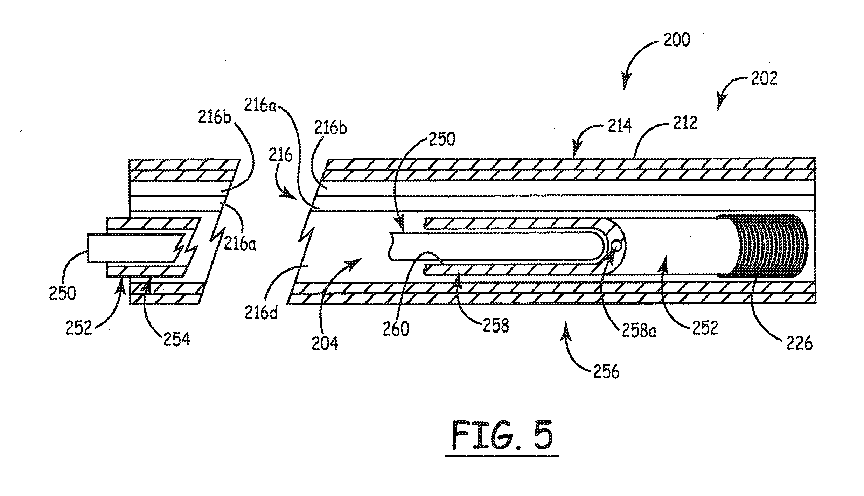

With reference now to FIG. 5, in one example, the confirmation member 204 could comprise a stylet 250, an elongated tubular body 252 and the tracking device 226. The stylet 250 can be used by the surgeon to guide the lead 202 into place within the anatomy. The stylet 250 can comprise any suitable stiffening device, and can be composed of a polymer, metal, metal alloy or combinations thereof. In one example, the stylet 250 can comprise a metallic member, such as a guide wire, which can be received into the elongated tubular body 252.

The elongated tubular body 252 can include a proximal end 254, a distal end 256, a wall 258 and can include a cannulated bore 260. In one example, the elongated tubular body 252 can comprise a polymeric tubing member, which can be comprised of any suitable polymeric material, such as polytetrafluoroethylene (PTFE), fluorinated ethylene propylene (FEP), perfluoroalkoxy (PFA), ethylene tetrafluoroethylene (ETFE), etc. The proximal end 254 can generally extend outside of the anatomical structure of the patient 12 when the confirmation member 204 is used during the surgical procedure. In some cases, the proximal end 254 can include a graspable portion, to enable the surgeon to manipulate or direct the movement of the distal end 256 of the confirmation member 204 within the anatomical structure. The distal end 256 can be opposed from the proximal end 254.

The wall 258 can couple the distal end 256 to the proximal end 254. The wall 258 can define a lumen 258a. In one example, at least a portion of the tracking device 226 can pass through the lumen 258a. For example, in the case of a wired electromagnetic tracking device 58a, the wires can pass through the lumen 258a to enable the tracking device 226 to communicate with the navigation probe interface 50. The cannulated bore 260 can be sized to receive the stylet 250. Generally, the cannulated bore 260 can be sized to enable the stylet 250 to be slidably received within the elongated tubular body 252. The tracking device 226 can be coupled to the distal end 256. As discussed, the tracking device 226 can provide a location and/or orientation of the elongated tubular body 252 in the patient space substantially in real-time, which can be used to map a location of the lead 202 within the anatomy.

The confirmation member 204 can be used by the surgeon to ensure that the lead 202 is properly positioned in the anatomy. In this regard, the confirmation member 204 can be inserted into the fourth conduit 216d, and the location of the confirmation member 204 within the fourth conduit 216d can be tracked by the navigation system 10 using the tracking device 226. If the confirmation member 204 does not include the stylet 250, then the confirmation member 204 can be inserted into the fourth conduit 216d. In this example, the flexible nature of the confirmation member 204 itself can enable the confirmation member 204 to cooperate with the navigation system 10 to map the shape and the location of the lead 202 within the anatomy.

In other words, the flexible nature of the confirmation member 204 can enable the confirmation member 204 to slide within the fourth conduit 216d without substantially altering the position or the shape of the lead 202 within the anatomy. Thus, by tracking the tracking device 226 of the confirmation member 204 within the fourth conduit 216d, the control module 101 can determine the position and the shape of the lead 202 within the anatomy. The position and the shape of the lead 202 can then be displayed on the display 36 as the icon 103 superimposed onto image data.

In the case of the confirmation member 204, which includes the stylet 250, the stylet 250 can be inserted into and coupled to the anatomy in the desired location. Then, the elongated tubular body 252 can be positioned over the stylet 250. The lead 202 can be positioned over the elongated tubular body 252 and the stylet 250, and coupled to the anatomy. Then, the stylet 250 can be removed or retracted from the anatomy, so that the elongated tubular body 252 can take the shape of the lead 202.

In this regard, as the stylet 250 is removed from the lead 202, the flexible nature of the elongated tubular body 252 can enable the elongated tubular body 252 to assume the shape and position of the lead 202. The elongated tubular body 252 can then be moved relative to the lead 202 to determine the location of the lead 202 within the anatomy using the tracking device 226. By tracking the tracking device 226 with the navigation system 10, the control module 101 can determine the position and the shape of the lead 202 with the anatomy, which can be displayed on the display 36 as the icon 103 superimposed onto the image data 102.

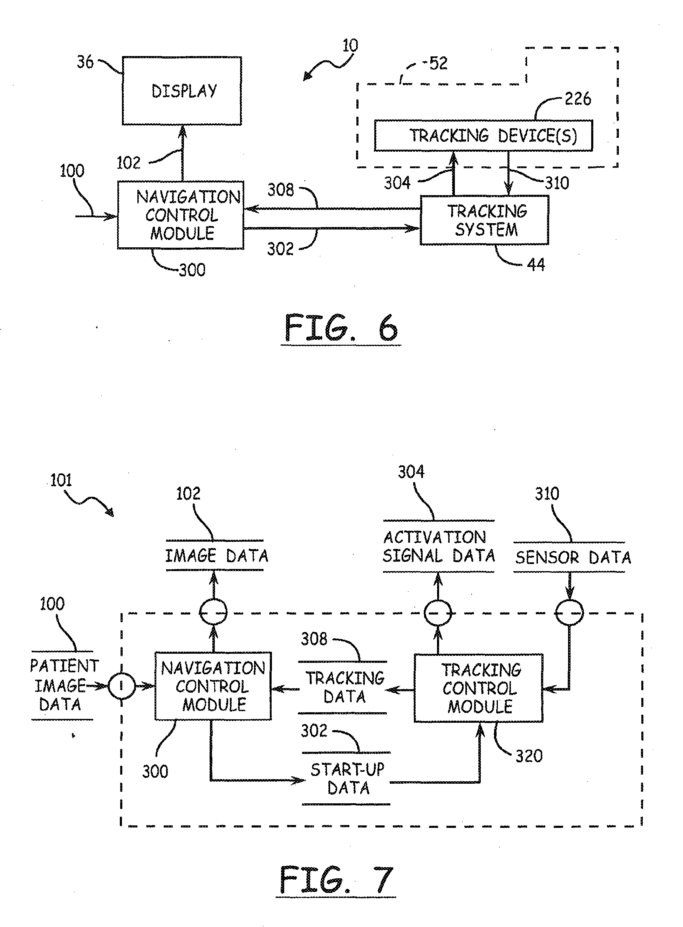

With reference now to FIG. 6, a simplified block diagram schematically illustrates an exemplary navigation system 10 for implementing the control module 101. The navigation system 10 can include the tracking system 44, the instrument 52, a navigation control module 300 and the display 36. The instrument 52 can include the tracking device(s) 226.

The tracking system 44 can comprise an electromagnetic tracking system 44 or an optical tracking system 44b, and will generally be referred to as the tracking system 44. The tracking system 44 can receive start-up data 302 from the navigation control module 300. In the case of an electromagnetic tracking system 44, based on the start-up data 302, the tracking system 44 can set activation signal data 304 that can activate the coil arrays 46, 47 to generate an electromagnetic field to which the tracking device(s) 226 coupled to the instrument 52, such as the confirmation member 204, can respond. The tracking system 44 can also set tracking data 308 for the navigation control module 300, as will be discussed. The tracking data 308 can include data regarding the coordinate position (location and orientation) of the tracking device(s) 226 coupled to the instrument 52, such as the confirmation member 204, in the patient space as computed from data received from the tracking device(s) 226.

When the tracking device(s) 226 are activated, the tracking device(s) 226 can transmit sensor data 310 indicative of a position of the tracking device 226 in the patient space to the tracking system 44. Based on the sensor data 310 received by the tracking system 44, the tracking system 44 can generate and set the tracking data 308 for the navigation control module 300.

The navigation control module 300 can receive the tracking data 308 from the tracking system 44 as input. The navigation control module 300 can also receive patient image data 100 as input. The patient image data 100 can comprise images of the anatomy of the patient 12 obtained from a pre- or intra-operative imaging device, such as the images obtained by the imaging device 14. Based on the tracking data 308 and the patient image data 100, the navigation control module 300 can generate image data 102 for display on the display 36. The image data 102 can comprise the patient image data 100 superimposed with an icon 103 of the instrument 52, such as the lead 202, with a substantially real-time indication of the position of the lead 202 in patient space, as shown in FIG. 1. The image data 102 could also comprise a schematic illustration of the lead 202 within the anatomy of the patient 12, etc.

With reference now to FIG. 7, a dataflow diagram illustrates an exemplary control system that can be embedded within the control module 101. Various embodiments of the control system according to the present disclosure can include any number of sub-modules embedded within the control module 101. The sub-modules shown may be combined and/or further partitioned to similarly determine the position of the lead 202 within the patient space based on the signals generated by the tracking device(s) 226. In various embodiments, the control module 101 includes the tracking system 44 that can implement a tracking control module 320 and the workstation 34 that can implement the navigation control module 300. It should be noted, however, that the tracking control module 320 and the navigation control module 300 could be implemented on the workstation 34, if desired.

The tracking control module 320 can receive as input the start-up data 302 from the navigation control module 300 and sensor data 310 from the tracking device(s) 226. Upon receipt of the start-up data 302, the tracking control module 320 can output the activation signal data 304 for the tracking device(s) 226. Upon receipt of the sensor data 310, the tracking control module 320 can set the tracking data 308 for the navigation control module 300. As discussed, the tracking data 308 can include data regarding the coordinate positions (locations and orientations) of the confirmation member 204.

The navigation control module 300 can receive as input the tracking data 308 and patient image data 100. Based on the tracking data 308, the navigation control module 300 can determine the appropriate patient image data 100 for display on the display 36, and can output both the tracking data 308 and the patient image data 100 as image data 102.

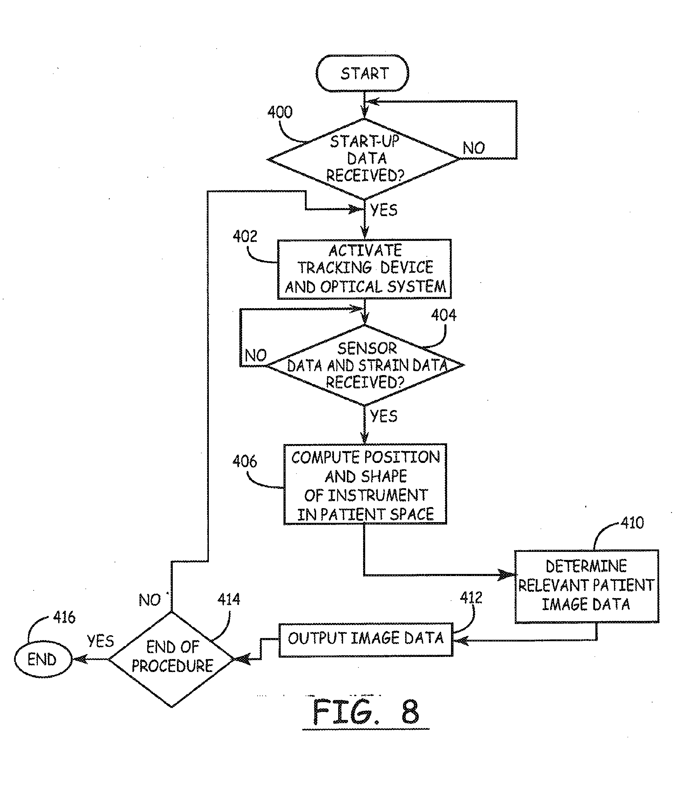

With reference now to FIG. 8, a flowchart diagram illustrates an exemplary method performed by the control module 101. At decision block 400, the method can determine if start-up data 302 has been received from the navigation control module 300. If no start-up data 302 has been received, then the method loops to decision block 400 until start-up data 302 is received. If start-up data 302 is received, then the method goes to block 402. At block 402, the tracking system 44 can generate the activation signal data 304. Then, at decision block 404 the method can determine if the sensor data 310 has been received. If the sensor data 310 has been received, then the method goes to block 406. Otherwise, the method loops to decision block 404 until the sensor data 310 is received.

At block 406, the method can compute the position of the lead 202 in patient space based on the sensor data 310. In this regard, the sensor data 310 can provide a position of the tracking device 226 in patient space. As the tracking device 226 is coupled to the confirmation member 204, and the confirmation member 204 is confined to move within the lead 202, the sensor data 310 can provide a position of the lead 202 in the patient space as the confirmation member 204 moves within the lead 202. At block 410, the method determine the relevant patient image data 100 for display on the display 36 based on the tracking data 308. Then, at block 412, the method can output the image data 102 that includes the icon 103 of the lead 202 superimposed on the patient image data 100 based on the patient image data 100 and the tracking data 308. At decision block 414, the method can determine if the surgical procedure has ended. If the surgical procedure has ended, then the method can end at 416. Otherwise, the method can loop to block 402.

Therefore, the instrument 52 of the present disclosure, for example, the confirmation member 204, can provide a user, such as a surgeon, with an accurate representation of the position and the shape of the lead 202 within the patient space during the surgical procedure. In this regard, the use of the tracking device 226 on the confirmation member 204 can enable the surgeon to move the confirmation member 204 within the lead 202 to map the position and the shape of the lead 202 within the anatomy, thereby providing an accurate depiction of the position and the shape of an elongated instrument, such as the lead 202, within the anatomical structure of the patient 12. Further, since the confirmation member 204 is traekable by the navigation system 10 and movable within the lead 202, the use of the confirmation member 204 with the navigation system 10 can enable the user to visualize the shape of the lead 202 from a proximal end to a distal end of the lead 202. Thus, the position and the shape of the lead 202 can be determined without the use of the imaging device 14.

While specific examples have been described in the specification and illustrated in the drawings, it will be understood by those of ordinary skill in the art that various changes may be made and equivalents may be substituted for elements thereof without departing from the scope of the present disclosure. Furthermore, the mixing and matching of features, elements and/or functions between various examples is expressly contemplated herein so that one of ordinary skill in the art would appreciate from this disclosure that features, elements and/or functions of one example may be incorporated into another example as appropriate, unless described otherwise, above. Moreover, many modifications may be made to adapt a particular situation or material to the teachings of the present disclosure without departing from the essential scope thereof. Therefore, it is intended that the present disclosure not be limited to the particular examples illustrated by the drawings and described in the specification as the best mode presently contemplated for carrying out this disclosure, but that the scope of the present disclosure will include any embodiments falling within the claims.

## Claims

1. A system for determining a location and a shape of a medical device within an anatomy comprising:
a medical instrument (52) having an elongate tubular body that defines at least one guide channel (220) for insertion into an anatomy;
a confirmation member (204) positioned within the at least one guide channel and movable therein relative to the medical instrument; the confirmation member comprising an elongated tubular body (252), the tubular body being comprised of a polymeric material and having a flexible nature to enable the body, when inserted within the guide channel, to move within the guide channel without substantially altering the position or shape of the medical instrument; and
at least one tracking device (226) coupled to the confirmation member;
a tracking system (44) that tracks a position of the at least one tracking device relative to the anatomy; and
a navigation system (10) that determines a position of the confirmation member relative to the anatomy based on the position of the at least one tracking device and determines a position and a shape of the medical instrument within the anatomy based on the position of the confirmation member.

2. The system of Claim 1, further comprising:
an imaging device (14) that acquires an image of the anatomical structure.

3. The system of Claim 2, further comprising:
a display (36) that displays the image of the anatomy superimposed with an icon of the medical instrument at a location that corresponds to the position of the medical instrument relative to the anatomical structure based on the position of the confirmation member.

4. The system of Claim 1, wherein the at least one tracking device further comprises at least one wired tracking device, and the confirmation member is cannulated to allow at least a portion of the wires associated with the at least one wired tracking device to pass from the distal end of the confirmation member to the proximal end of the confirmation member.

5. The system of Claim 1, wherein the confirmation member further comprises:
a flexible tubular member having a wall that defines a lumen and a bore that each extend from the proximal end of the confirmation member to the distal end of the confirmation member; and
a rigid stylet (250) that is movable within the bore from a first position to a second position.

6. The system of Claim 5, wherein in the second position, the rigid stylet is moved a distance from the distal end of the bore so that the distal end of the confirmation member is unsupported within the medical instrument.

7. The system of Claim 5, wherein in the second position, the confirmation member assumes the shape of the medical instrument so that the movement of the confirmation member relative to the medical instrument enables the navigation system to determine the position of the medical instrument within the anatomy.

8. The system of Claim 1, wherein the at least one tracking device comprises at least one electromagnetic tracking device selected from the group including: an electromagnetic receiver tracking device, an electromagnetic transmitter tracking device and combinations thereof.

9. The system of any preceding claim, wherein said medical instrument is a cardiac lead.

## Patentansprüche

1. System zum Bestimmen eines Ortes und einer Form einer medizinischen Vorrichtung in einer Anatomie, das enthält:
ein medizinisches Instrument (52) mit einem langgestreckten rohrförmigen Körper, der wenigstens einen Führungskanal (220) für die Einführung in eine Anatomie definiert;
ein Bestätigungselement (204), das in dem wenigstens einen Führungskanal positioniert ist und darin relativ zu dem medizinischen Instrument beweglich ist; wobei das Bestätigungselement einen langgestreckten rohrförmigen Körper (252) aufweist, wobei der rohrförmige Körper ein Polymermaterial enthält und flexibel ist, um dem Körper zu ermöglichen, sich dann, wenn er in den Führungskanal eingeführt ist, in dem Führungskanal zu bewegen, ohne die Position oder die Form des medizinischen Instruments wesentlich zu verändern; und
wenigstens eine Nachverfolgungsvorrichtung (226), die mit dem Bestätigungselement gekoppelt ist;
ein Nachverfolgungssystem (44), das eine Position der wenigstens einen Nachverfolgungsvorrichtung relativ zu der Anatomie nachverfolgt; und
ein Navigationssystem (10), das eine Position des Bestätigungselements relativ zu der Anatomie anhand der Position der wenigstens einen Nachverfolgungsvorrichtung bestimmt und eine Position und eine Form des medizinischen Instruments in der Anatomie anhand der Position des Bestätigungselements bestimmt.

2. System nach Anspruch 1, das ferner enthält:
eine Bildgebungsvorrichtung (14), die ein Bild der anatomischen Struktur erfasst.

3. System nach Anspruch 2, das ferner enthält:
eine Anzeige (36), die anhand der Position des Bestätigungselements das Bild der Anatomie überlagert mit einem Icon des medizinischen Instruments an einem Ort, der der Position des medizinischen Instruments relativ zu der anatomischen Struktur entspricht, anzeigt.

4. System nach Anspruch 1, wobei die wenigstens eine Nachverfolgungsvorrichtung ferner wenigstens eine drahtgebundene Nachverfolgungsvorrichtung enthält und das Bestätigungselement kanüliert ist, um wenigstens einem Abschnitt der Drähte, die der wenigstens einen drahtgebundenen Nachverfolgungsvorrichtung zugeordnet sind, zu ermöglichen, sich von dem distalen Ende des Bestätigungselements zu dem proximalen Ende des Bestätigungselements zu bewegen.

5. System nach Anspruch 1, wobei das Bestätigungselement ferner enthält:
ein biegsames rohrförmiges Element, das eine Wand, die ein Lumen definiert, und eine Bohrung besitzt, die sich jeweils von dem proximalen Ende des Bestätigungselements zu dem distalen Ende des Bestätigungselements erstrecken; und
ein starres Stilett (250), das in der Bohrung von einer ersten Position zu einer zweiten Position beweglich ist.

6. System nach Anspruch 5, wobei das starre Stilett in der zweiten Position um eine Strecke von dem distalen Ende der Bohrung bewegt ist, so dass das distale Ende des Bestätigungselements in dem medizinischen Instrument nicht unterstützt ist.

7. System nach Anspruch 5, wobei das Bestätigungselement in der zweiten Position die Form des medizinischen Instruments annimmt, so dass die Bewegung des Bestätigungselements relativ zu dem medizinischen Instrument dem Navigationssystem ermöglicht, die Position des medizinischen Instruments in der Anatomie zu bestimmen.

8. System nach Anspruch 1, wobei die wenigstens eine Nachverfolgungsvorrichtung wenigstens eine elektromagnetische Nachverfolgungsvorrichtung enthält, die aus der Gruppe ausgewählt ist, die enthält: eine elektromagnetische Empfänger-Nachverfolgungsvorrichtung, eine elektromagnetische Sender-Nachverfolgungsvorrichtung und Kombinationen hiervon.

9. System nach einem vorhergehenden Anspruch, wobei das medizinische Instrument eine kardiale Leitung ist.

## Revendications

1. Système pour déterminer un emplacement et une forme d'un dispositif médical à l'intérieur d'une anatomie, comportant :
un instrument médical (52) ayant un corps tubulaire allongé qui définit au moins un canal de guidage (220) en vue d'une insertion dans une anatomie ;
un élément de confirmation (204) positionné à l'intérieur du au moins un canal de guidage et mobile dans celui-ci par rapport à l'instrument médical ; l'élément de confirmation comportant un corps tubulaire allongé (250), le corps tubulaire étant constitué d'une matière polymère et ayant une nature souple pour permettre au corps, lorsqu'il est inséré à l'intérieur du canal de guidage, de se déplacer à l'intérieur du canal de guidage sans modifier sensiblement la position ou la forme de l'instrument médical ; et
au moins un dispositif de suivi (226) couplé à l'élément de confirmation ;
un système de suivi (44) qui suit une position du au moins un dispositif de suivi par rapport à l'anatomie ; et
un système de navigation (10) qui détermine une position de l'élément de confirmation par rapport à l'anatomie sur la base de la position du au moins un dispositif de suivi et
détermine une position et une forme de l'instrument médical à l'intérieur de l'anatomie sur la base de la position de l'élément de confirmation.

2. Système selon la revendication 1, comportant en outre :
un dispositif d'imagerie (14) qui acquiert une image de la structure anatomique.

3. Système selon la revendication 2, comportant en outre :
un affichage (36) qui affiche l'image de l'anatomie superposée avec une icône de l'instrument médical à un emplacement qui correspond à la position de l'instrument médical par rapport à la structure anatomique sur la base de la position de l'élément de confirmation.

4. Système selon la revendication 1, dans lequel le au moins un dispositif de suivi comporte en outre au moins un dispositif de suivi filaire, et l'élément de confirmation est canulé pour permettre à au moins une portion des fils associés au au moins un dispositif de suivi filaire de passer de l'extrémité distale de l'élément de confirmation à l'extrémité proximale de l'élément de confirmation.

5. Système selon la revendication 1, dans lequel l'élément de confirmation comporte en outre :
un élément tubulaire souple ayant une paroi qui définit une lumière et un alésage qui s'étendent chacun depuis l'extrémité proximale de l'élément de confirmation jusqu'à l'extrémité distale de l'élément de confirmation ; et
un stylet rigide (250) qui est mobile à l'intérieur de l'alésage d'une première position à une seconde position.

6. Système selon la revendication 5, dans lequel dans la seconde position, le stylet rigide est déplacé sur une certaine distance à partir de l'extrémité distale de l'alésage de telle sorte que l'extrémité distale de l'élément de confirmation n'est pas supportée à l'intérieur de l'instrument médical.

7. Système selon la revendication 5, dans lequel dans la seconde position, l'élément de confirmation prend la forme de l'instrument médical de sorte que le mouvement de l'élément de confirmation par rapport à l'instrument médical permet au système de navigation de déterminer la position de l'instrument médical à l'intérieur de l'anatomie.

8. Système selon la revendication 1, dans lequel le au moins un dispositif de suivi comporte au moins un dispositif de suivi électromagnétique choisi parmi le groupe incluant : un dispositif de suivi à récepteur électromagnétique, un dispositif de suivi à émetteur électromagnétique et des combinaisons de ceux-ci.

9. Système selon l'une quelconque des revendications précédentes, dans lequel ledit instrument médical est une sonde cardiaque.
